# EUROPEAN PATENT APPLICATION

(11) **EP 4 667 835 A1**
(43) Date of publication of application: **24.12.2025**
(21) Application number: 24811328.4
(22) Date of filing: 13.05.2024
(51) Int. Cl.: F24F 1/0071, F24F 8/22, F24F 13/10, F24F 13/20, F24F 11/70, F24F 11/89, A61L 2/10, A61L 9/20, F24F 120/12

(54) **AIR CONDITIONER**

(30) Priority: 24.05.2023 KR 20230066848; 18.08.2023 KR 20230108551
(71) Applicant: Samsung Electronics Co., Ltd., Suwon-si, Gyeonggi-do 16677 (KR)
(72) Inventor: YOON, Joonho, Suwon-si, Gyeonggi-do 16677 (KR); KIM, Hyunho, Suwon-si, Gyeonggi-do 16677 (KR); PARK, Wooyoung, Suwon-si, Gyeonggi-do 16677 (KR); SO, Byungyul, Suwon-si, Gyeonggi-do 16677 (KR); SHIN, Moonsun, Suwon-si, Gyeonggi-do 16677 (KR); AHN, Soonwon, Suwon-si, Gyeonggi-do 16677 (KR); YOO, Byeongkook, Suwon-si, Gyeonggi-do 16677 (KR); LEE, Hojin, Suwon-si, Gyeonggi-do 16677 (KR); JEON, Mingu, Suwon-si, Gyeonggi-do 16677 (KR); JUNG, Changwoo, Suwon-si, Gyeonggi-do 16677 (KR)
(74) Representative: Walaski, Jan Filip
(86) International application number: PCT/KR2024/006508
(87) International publication number: WO 2024/242389

(57) **Abstract**

An air conditioner includes: a housing including an air inlet and an air outlet, a heat exchanger provided inside the housing, a blower fan rotatably provided inside the housing, an ultraviolet (UV) light source provided inside the housing and configured to irradiate UV light toward the blower fan, a blade rotatable, relative to the housing, configured to be in an open position for opening the air outlet or in a closed position for blocking the air outlet, and a sensor configured to detect a position of the blade, and may be configured to selectively stop operation of the UV light source based on the position of the blade detected by the sensor.

## Description

### Technical Field

Embodiments of the disclosure relate to an air conditioner, and more particularly, to an air conditioner having an ultraviolet light source placed therein.

### Background Art

An air conditioner is an apparatus that performs functions such as air purification, ventilation, humidity control, cooling, or heating in an air-conditioned space, such as an indoor space. An air conditioner may include a refrigeration cycle in which refrigerant is circulated to perform a cooling or heating function.

Air conditioners may be classified into a ceiling-mounted air conditioner, a stand-type air conditioner, a wall-mounted air conditioner, etc., depending on installation locations thereof. A ceiling-mounted air conditioner is a type of air conditioner that is installed on the ceiling, a wall-mounted air conditioner is a type of air conditioner that is installed by being attached to a wall, and a stand-type air conditioner is a type of air conditioner that is installed standing upright in an indoor space.

An ultraviolet light source for sterilization may be placed inside these air conditioners.

### Disclosure

### Technical Solution

According to an example embodiment of the disclosure, an air conditioner includes: a housing having an air inlet and an air outlet, a heat exchanger provided inside the housing, a blower fan rotatably provided inside the housing, a ultraviolet (UV) light source provided inside the housing and configured to irradiate UV light toward the blower fan, and a blade rotatable, relative to the housing, and configured to be in an open position (e.g., pose) for opening the air outlet or in a closed position for blocking the air outlet.

The air conditioner may further include a sensor configured to detect a position of the blade.

The air conditioner may selectively stop operation of the UV light source based on the position of the blade detected by the sensor.

According to an example embodiment of the disclosure, an air conditioner includes: a housing having an air inlet and an air outlet, a heat exchanger provided inside the housing, a blower fan rotatably provided inside the housing, a UV light source provided inside the housing and configured to irradiate UV light toward the blower fan, and a blade rotatable relative to the housing and configured to be in an open position for opening the air outlet or in a closed position for blocking the air outlet.

The air conditioner may further include a sensor configured to detect whether the air outlet is accessible to a user.

The air conditioner may be configured to control operation of the UV light source based on a result of the detection by the sensor.

### Description of Drawings

FIG. 1 is a perspective view of an air conditioner according to various embodiments;
FIG. 2 is an exploded perspective view of an air conditioner according to various embodiments;
FIG. 3 is a cross-sectional view of an air conditioner according to various embodiments;
FIG. 4 is a perspective view illustrating a blower fan and a ultraviolet (UV) light source in an air conditioner, according to various embodiments;
FIG. 5 is a partial cross-sectional view illustrating irradiation of a blower fan with UV light by a UV light source in an air conditioner, according to various embodiments;
FIG. 6 is a partial cross-sectional view illustrating a state in which operation of a UV light source of an air conditioner is stopped, according to various embodiments;
FIG. 7 is a flowchart illustrating example operation of a UV light source of an air conditioner, according to various embodiments;
FIG. 8 is a perspective view illustrating an example of a sensor of an air conditioner, according to various embodiments;
FIG. 9 is a cross-sectional view illustrating an operating state of a UV light source using the sensor of FIG. 8 according to various embodiments;
FIG. 10 is a cross-sectional view illustrating an operating state of a UV light source using the sensor of FIG. 8 according to various embodiments;
FIG. 11 is a cross-sectional view illustrating another example of a sensor of an air conditioner, according to various embodiments;
FIG. 12 is a cross-sectional view illustrating another example of a sensor of an air conditioner, according to various embodiments;
FIG. 13 is a perspective view illustrating another example of a sensor of an air conditioner, according to various embodiments;
FIG. 14 is a perspective view illustrating various example components surrounding an air outlet of an air conditioner, according to various embodiments;
FIG. 15 is a cross-sectional view illustrating an example in which air is discharged to the outside when a blade is in a closed position in an air conditioner, according to various embodiments;
FIG. 16 is a flowchart illustrating example operations for selectively operating a UV light source in an air conditioner, according to various embodiments;
FIG. 17 is a diagram illustrating whether a UV light source of an air conditioner is operating, according to various embodiments;
FIG. 18 is a diagram illustrating whether a UV light source of an air conditioner is operating, according to various embodiments;
FIG. 19 is a diagram illustrating a reference distance between a user and a sensor in an air conditioner, according to various embodiments;
FIG. 20 is a cross-sectional view illustrating an example of an air conditioner according to various embodiments;
FIG. 21 is a cross-sectional view illustrating an example operation of the air conditioner of FIG. 20 according to various embodiments;
FIG. 22 is a cross-sectional view illustrating an example of an air conditioner according to various embodiments;
FIG. 23 is a cross-sectional view illustrating an example operation of the air conditioner of FIG. 22 according to various embodiments;
FIG. 24 is a perspective view illustrating another example of an air conditioner according to various embodiments;
FIG. 25 is an exploded perspective view illustrating another example of an air conditioner according to various embodiments; and
FIG. 26 is a perspective view illustrating another example of an air conditioner according to various embodiments.

### Mode for Invention

It should be understood that various example embodiments of the disclosure and terms used herein are not intended to limit the technical features described herein to particular embodiments of the disclosure and that the disclosure includes various modifications, equivalents, or substitutions of various embodiments of the disclosure.

With regard to the description of the drawings, like reference numerals may be used to represent like or related elements.

A singular form of a noun corresponding to an item may include one or a plurality of the items unless the context clearly indicates otherwise.

As used herein, each of the phrases such as "A or B," "at least one of A and B, "at least one of A or B," "A, B, or C," "at least one of A, B, and C," and "at least one of A, B, or C" may include any one of the items listed together in a corresponding one of the phrases, or all possible combinations thereof.

The term "and/or" includes any combination of a plurality of associated elements listed, or any one of the plurality of associated listed elements.

Terms such as "first," "second," etc. may be used simply to distinguish an element from other elements and do not limit the elements in any other respect (e.g., importance or order).

It will be understood that when an element (e.g., a first element) is referred to, with or without the term "functionally" or "communicatively", as being "coupled" or "connected" to another element (e.g., a second element), the element may be coupled to the other element directly (e.g., in a wired manner), wirelessly, or via a third element.

The terms such as "comprise," "include," or "have" are intended to specify the presence of stated features, numbers, steps, operations, elements, components, or combinations thereof, but do not preclude the presence or addition of one or more other features, numbers, steps, operations, elements, components, or combinations thereof.

It will also be understood that when an element is referred to as being "connected," "coupled," "supported," or "in contact" with another element, this includes not only when the elements are directly connected, coupled, supported, or in contact, but also when they are indirectly connected, coupled, supported, or in contact via a third element.

It will also be understood that when an element is referred to as being "on" another element, the element may be directly on the other element, or intervening elements may also be present therebetween.

An air conditioner according to various embodiments of the disclosure may refer to an apparatus that performs functions such as air purification, ventilation, humidity control, cooling, or heating in an air-conditioned space (hereinafter referred to as an "indoor space") and is equipped with at least one of these functions.

According to an embodiment of the disclosure, an air conditioner may include a heat pump system to perform a cooling function or a heating function. The heat pump system may include a refrigeration cycle in which a refrigerant is circulated through a compressor, a first heat exchanger, an expansion device, and a second heat exchanger. All components of a heat pump system may be built into a single housing that forms an external appearance of an air conditioner, and window-type air conditioners or portable air conditioners are examples of such an air conditioner. On the other hand, components of the heat pump system may be split into several parts and built into a plurality of housings that form a single air conditioner, and examples of such an air conditioner include wall-mounted air conditioners, stand-type air conditioners, and system air conditioners.

An air conditioner including a plurality of housings may include at least one outdoor unit installed outdoors and at least one indoor unit installed indoors. For example, an air conditioner may be provided with one outdoor unit and one indoor unit connected via a refrigerant pipe. For example, an air conditioner may include one outdoor unit and two or more indoor units connected via a refrigerant pipe. For example, an air conditioner may include two or more outdoor units and two or more indoor units connected via a plurality of refrigerant pipes.

An outdoor unit may be electrically connected to an indoor unit. For example, information (or commands) for controlling an air conditioner may be input via an input interface provided on the outdoor or indoor unit, and the outdoor unit and the indoor unit may operate simultaneously or sequentially in response to a user input.

The air conditioner may include an outdoor heat exchanger provided in an outdoor unit, an indoor heat exchanger provided in an indoor unit, and a refrigerant pipe connecting the outdoor heat exchanger to the indoor heat exchanger.

The outdoor heat exchanger may exchange heat between a refrigerant and outdoor air using a phase change (e.g., evaporation or condensation) of the refrigerant. For example, the refrigerant may release heat into the outdoor air during condensation of the refrigerant in the outside heat exchanger, and the refrigerant may absorb heat from the outdoor air during evaporation of the refrigerant flowing in the outside heat exchanger.

The indoor unit is installed indoors. For example, indoor units may be classified into ceiling-mounted indoor units, stand-type indoor units, wall-mounted indoor units, etc., depending on how they are arranged. For example, ceiling-mounted indoor units may be subdivided into 4-way cassette indoor units, 1-way cassette indoor units, duct-type indoor units, etc. depending on a way air is discharged.

Similarly, the indoor heat exchanger may exchange heat between a refrigerant and indoor air using a phase change (e.g., evaporation or condensation) of the refrigerant. For example, while the refrigerant evaporates in the indoor heat exchanger, the refrigerant may absorb heat from the indoor air, and the indoor air is cooled as it passes through the cold indoor heat exchanger and then blown out to cool an indoor space. Furthermore, while a refrigerant condenses in the indoor heat exchanger, the refrigerant may release heat into the indoor air, and the indoor air is heated as it passes through the high-temperature indoor heat exchanger and then blown out to heat the indoor space.

For example, the air conditioner performs a cooling or heating function through a phase change process undergone by the refrigerant circulating between the outdoor heat exchanger and the indoor heat exchanger, and for this circulation of the refrigerant, the air conditioner may include a compressor that compresses the refrigerant. The compressor may suck in refrigerant gas through a suction port and compress the refrigerant gas. The compressor may discharge high-temperature, high-pressure refrigerant gas via a discharge port. The compressor may be placed inside the outdoor unit.

The refrigerant may circulate, via a refrigerant pipe, through the compressor, the outdoor heat exchanger, the expansion device, and the indoor heat exchanger in the stated order, or through the compressor, the indoor heat exchanger, the expansion device, and the outdoor heat exchanger in the stated order.

For example, when the air conditioner has one outdoor unit and one indoor unit directly connected via a refrigerant pipe, the refrigerant may circulate between the one outdoor unit and the one indoor unit through the refrigerant pipe.

For example, when the air conditioner has one outdoor unit connected to two or more indoor units via a refrigerant pipe, refrigerants may flow into the plurality of indoor units via refrigerant pipes branching from the outdoor unit. The refrigerants discharged from the plurality of indoor units may be combined together and circulated to the outdoor unit. For example, the plurality of indoor units may each be directly connected to the one outdoor unit in parallel via separate refrigerant pipes.

Each of the plurality of indoor units may operate independently according to an operating mode set by a user. That is, some of the plurality of indoor units may operate in a cooling mode, and others may operate in a heating mode simultaneously. In this case, the refrigerant may be selectively introduced into each indoor unit at a high or low pressure along a designated circulation path via a flow path diverter valve as described below, and then discharged from the indoor unit and circulated to the outdoor unit.

For example, when the air conditioner has two or more outdoor units and two or more indoor units connected via a plurality of refrigerant pipes, refrigerants discharged from the plurality of outdoor units are combined and flow through a single refrigerant pipe, and then diverge again at a certain point to enter the plurality of indoor units.

The plurality of outdoor units may all be driven, or at least some of the outdoor units may not be driven, depending on an operating load corresponding to the amount of operation of the plurality of indoor units. In this case, the refrigerant may flow into and circulate through an outdoor unit that is selectively driven via a flow path diverter valve. The air conditioner may include an expansion device to lower the pressure of the refrigerant entering a heat exchanger. For example, the expansion device may be placed inside an indoor unit, inside an outdoor unit, or both.

For example, the expansion device may lower the temperature and pressure of the refrigerant using a throttling effect. The expansion device may include an orifice capable of reducing a cross-sectional area of a flow path. The temperature and pressure of the refrigerant that passes through the orifice may be lowered.

For example, the expansion device may be implemented as an electronic expansion valve capable of adjusting an opening ratio (a ratio of a cross-sectional area of a flow path in a valve in a partially open state to a cross-sectional area of a flow path in the valve in a fully open state). The flow rate of refrigerant passing through the expansion device may be controlled depending on the opening ratio of the electronic expansion valve.

The air conditioner may further include a flow path diverter valve provided on a refrigerant circulation flow path. The flow path diverter valve may include, for example, a 4-way valve. The flow path diverter valve may determine a path of circulation of the refrigerant depending on an operating mode of an indoor unit (e.g., cooling operation or heating operation). The flow path diverter valve may be connected to the discharge port of the compressor.

The air conditioner may include an accumulator. The accumulator may be connected to the suction port of the compressor. A low-temperature, low-pressure refrigerant evaporated from the indoor heat exchanger or outdoor heat exchanger may flow into the accumulator.

When a mixture of refrigerant liquid and refrigerant gas flows into the accumulator, the accumulator may separate the refrigerant liquid from the refrigerant gas and provide the refrigerant gas from which the refrigerant liquid has been separated to the compressor.

An outdoor fan may be provided in the vicinity of the outdoor heat exchanger. The outdoor fan may blow outdoor air into the outdoor heat exchanger to facilitate heat exchange between the refrigerant and the outdoor air.

An outdoor unit of the air conditioner may include at least one sensor. For example, an outdoor unit sensor may be provided as an ambient sensor. The outdoor unit sensor may be placed at any location on the inside or outside of the outdoor unit. The outdoor unit sensor may include, for example, a temperature sensor for detecting air temperature around the outdoor unit, a humidity sensor for detecting humidity in the air around the outdoor unit, a refrigerant temperature sensor for detecting a refrigerant temperature inside a refrigerant pipe passing through the outdoor unit, or a refrigerant pressure sensor for detecting a refrigerant pressure inside the refrigerant pipe passing through the outdoor unit.

The outdoor unit of the air conditioner may include an outdoor unit communication interface. The outdoor unit communication interface may be provided to receive a control signal from an indoor unit controller of the air conditioner, as described below. The outdoor unit may control, based on a control signal received via the outdoor unit communication interface, an operation of a compressor, an outdoor heat exchanger, an expansion device, a flow path diverter valve, an accumulator, or an outdoor fan. The outdoor unit may transmit, via the outdoor unit communication interface, a sensing value detected by the outdoor unit sensor to the indoor unit controller.

An indoor unit of the air conditioner may include a housing, a blower fan that circulates air inside or outside the housing, and an indoor heat exchanger that exchanges heat with air flowing into the housing.

The housing may include an air inlet. Indoor air may be drawn into the housing via the air inlet.

The indoor unit of the air conditioner may include a filter provided to filter out foreign substances from the air drawn into the housing via the air inlet.

The housing may include an air outlet. Air flowing inside the housing may be discharged from the housing via the air outlet.

The housing of the indoor unit may include an airflow guide that guides a direction of air discharged through the air outlet. For example, the airflow guide may include a blade located on the air outlet. For example, the airflow guide may include an auxiliary fan for regulating the discharged airflow. However, the disclosure is not limited thereto, and the airflow guide may be omitted.

Inside the housing of the indoor unit, the indoor heat exchanger and the blower fan may be provided on a flow path connecting the air inlet and the air outlet.

The blower fan may include an indoor fan and a fan motor. For example, indoor fans may include an axial fan, a diagonal fan, a crossflow fan, and a centrifugal fan.

The indoor heat exchanger may be placed between the blower fan and the air outlet, or between the air inlet and the blower fan. The indoor heat exchanger may absorb heat from air drawn in through the air inlet or transfer heat to the air drawn in through the air inlet. The indoor heat exchanger may include a heat exchange tube in which a refrigerant flows, and heat exchange fins that are in contact with the heat exchange tube to increase a heat transfer area.

The indoor unit of the air conditioner may include a drain tray located below the indoor heat exchanger to collect condensate water generated in the indoor heat exchanger. The condensate water collected in the drain tray may be drained to the outside via a drain hose. The drain tray may be provided to support the indoor heat exchanger.

The indoor unit of the air conditioner may include an input interface. The input interface may include any type of user input devices, including buttons, switches, touch screens, and/or touch pads. The user may directly input setting data (e.g., desired indoor temperature, operating mode settings for cooling/heating/dehumidification/air purification, outlet selection settings, and/or air volume settings) via the input interface.

The input interface may be connected to an external input device. For example, the input interface may be electrically connected to a wired remote controller. The wired remote controller may be installed at a specific location in an indoor space (e.g., a portion of a wall). The user may operate the wired remote controller to input setting data regarding an operation of the air conditioner. An electrical signal corresponding to the setting data obtained via the wired remote controller may be transmitted to the input interface. In addition, the input interface may include an infrared sensor. The user may remotely input setting data regarding the operation of the air conditioner using a wireless remote controller. The setting data input via the wireless remote controller may be transmitted to the input interface as an infrared signal.

The input interface may include a microphone. A user's voice command may be obtained via the microphone. The microphone may convert the user's voice command into an electrical signal and transmit the electrical signal to the indoor unit controller. The indoor unit controller may control components of the air conditioner to perform a function corresponding to the user's voice command. Setting data (e.g., desired indoor temperature, operating mode settings for cooling/heating/dehumidification/air purification, outlet selection settings, and/or air volume settings) obtained via the input interface may be transmitted to the indoor unit controller as described below. For example, the setting data obtained via the input interface may be transmitted to the outside, e.g., an outdoor unit or a server, via an indoor unit communication interface as described below.

The indoor unit of the air conditioner may include a power module. The power module may be connected to an external power source to supply power to components of the indoor unit.

The indoor unit of the air conditioner may include an indoor unit sensor. The indoor unit sensor may be an ambient sensor placed inside or outside the housing. For example, the indoor unit sensor may include one or more temperature sensors and/or one or more humidity sensors arranged in a predetermined space inside or outside the housing of the indoor unit. For example, the indoor unit sensor may include a refrigerant temperature sensor for detecting a refrigerant temperature inside a refrigerant pipe passing through the indoor unit. For example, the indoor unit sensor may include refrigerant temperature sensors that respectively detect temperatures at an inlet, a middle, and/or an outlet of the refrigerant pipe passing through the indoor heat exchanger.

For example, pieces of environment information respectively detected by the indoor unit sensors may be transmitted to the indoor unit controller as described below, or may be transmitted to the outside via the indoor unit communication interface as described below.

The indoor unit of the air conditioner may include the indoor unit communication interface comprising various communication circuitry. The indoor unit communication interface may include at least one of a short-range communication module or a long-range communication module. The indoor unit communication interface may include at least one antenna for wirelessly communicating with other devices. The outdoor unit may include the outdoor unit communication interface. The outdoor unit communication interface may also include at least one of a short-range communication module or a long-range communication module.

The short-range communication module may include various communication circuitry and may include, but is not limited to, a Bluetooth communication module, a Bluetooth Low Energy (BLE) communication module, a near field communication (NFC) communication module, a wireless local area network (WLAN) (or Wi-Fi) communication module, a ZigBee communication module, an Infrared Data Association (IrDA) communication module, a Wi-Fi Direct (WFD) communication module, an ultra-wideband (UWB) communication module, an Ant+ communication module, a microwave (uWave) communication module, etc.

The long-range communication module may include various communication circuitry and include a communication module that performs various types of long-range communications, and include a mobile communication interface. The mobile communication interface transmits or receives a wireless signal to or from at least one of a base station, an external terminal, or a server on a mobile communication network.

The indoor unit communication interface may include various circuitry and communicate with an external device such as a server, a mobile device, or another home appliance via a nearby access point (AP). The AP may connect a LAN to which the air conditioner or a user device is connected to a wide area network (WAN) to which a server is connected. The air conditioner or user device may be connected to the server via the WAN. The indoor unit of the air conditioner may include the indoor unit controller that controls the components of the indoor unit, including the blower fan, etc. The outdoor unit of the air conditioner may include an outdoor unit controller that controls components of the outdoor unit, including the compressor, etc. The indoor unit controller may communicate with the outdoor unit controller via the indoor unit communication interface and the outdoor unit communication interface. The outdoor unit communication interface may transmit a control signal generated by the outdoor unit controller to the indoor unit communication interface, or transmit, to the outdoor unit controller, a control signal transmitted from the indoor unit communication interface. In other words, the outdoor unit and the indoor unit may communicate in both directions. The outdoor unit and the indoor unit may transmit and receive various signals generated during an operation of the air conditioner.

The outdoor unit controller may include various processing and/or control circuitry and be electrically connected to the components of the outdoor unit and control operation of each of the components. For example, the outdoor unit controller may adjust a frequency of the compressor and control a flow path diverter valve to change a circulation direction of a refrigerant. The outdoor unit controller may adjust a rotation speed of the outdoor fan. In addition, the outdoor unit controller may generate a control signal for adjusting the degree of opening of an expansion valve. Under the control of the outdoor unit controller, the refrigerant may circulate along a refrigerant circulation circuit including the compressor, the flow path diverter valve, the outdoor heat exchanger, the expansion valve, and the indoor heat exchanger.

Various temperature sensors included in the outdoor unit and the indoor unit may each transmit an electrical signal corresponding to a temperature detected by each of the temperature sensors to the outdoor unit controller and/or the indoor unit controller. For example, each of humidity sensors included in the outdoor unit and the indoor unit may transmit an electrical signal corresponding to its detected humidity to the outdoor unit controller and/or the indoor unit controller.

The indoor unit controller may obtain a user input from a user device including a mobile device or the like via the indoor unit communication interface, and obtain a user input directly via the input interface or through a remote controller. The indoor unit controller may control the components of the indoor unit, including the blower fan, etc., in response to the received user input. The indoor unit controller may transmit information about the received user input to the outdoor unit controller of the outdoor unit.

The outdoor unit controller may include various processing and/or control circuitry and control the components of the outdoor unit, including the compressor, etc., based on information about a user input received from the indoor unit. For example, when a control signal corresponding to a user input for selecting an operating mode such as cooling operation, heating operation, blowing operation, defrosting operation, or dehumidifying operation is received from the indoor unit, the outdoor unit controller may control the components of the outdoor unit to perform an operation of the air conditioner, corresponding to the selected operating mode.

The outdoor unit controller and the indoor unit controller may each include a processor and memory. The indoor unit controller may include at least one first processor and at least one first memory, and the outdoor unit controller may include at least one second processor and at least one second memory.

A memory may record/store various pieces of information necessary for operations of the air conditioner. The memory may store instructions, applications, data, and/or programs necessary for operations of the air conditioner. For example, the memory may store various programs for a cooling operation, a heating operation, a dehumidifying operation, and/or a defrosting operation of the air conditioner. The memory may include volatile memories, such as static random access memory (SRAM) and dynamic RAM (DRAM), for temporarily storing data. Furthermore, the memory may include non-volatile memories for long-term storage of data, such as read-only memory (ROM), erasable programmable ROM (EPROM), and electrically erasable PROM (EEPROM).

A processor may include various processing circuitry and generate control signals for controlling operations of the air conditioner, based on instructions, applications, data, and/or programs stored in the memory. The processor is a hardware component and may include logic circuits and arithmetic circuits. The processor may process data according to programs and/or instructions provided from the memory and generate control signals based on processing results. The memory and the processor may each be implemented as a single control circuit or as a plurality of circuits. For example, the processor according to an embodiment of the disclosure may include various processing circuitry and/or multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions.

The indoor unit of the air conditioner may include an output interface. The output interface is electrically connected to the indoor unit controller and may output information related to an operation of the air conditioner under the control of the indoor unit controller. For example, the output interface may output information such as operating mode, wind direction, air volume, and temperature selected via a user input. In addition, the output interface may output sensing information and warning/error messages obtained from the indoor unit sensor or the outdoor unit sensor.

The output interface may include a display and a speaker. The speaker is an audio device that may output a variety of sounds. The display may display information input by the user or information provided to the user using various graphical elements. For example, operation information about the air conditioner may be displayed as at least one of an image or text. The display may also include indicators that provide specific information. The display may include a liquid crystal display (LCD) panel, a light emitting diode (LED) panel, an organic light emitting diode (OLED) panel, a micro LED panel, and/or a plurality of LEDs.

Hereinafter, indoor units of air conditioners according to various embodiments of the disclosure are described in greater detail with reference to the drawings. For convenience of description, an indoor unit of an air conditioner may hereinafter be referred to as an air conditioner.

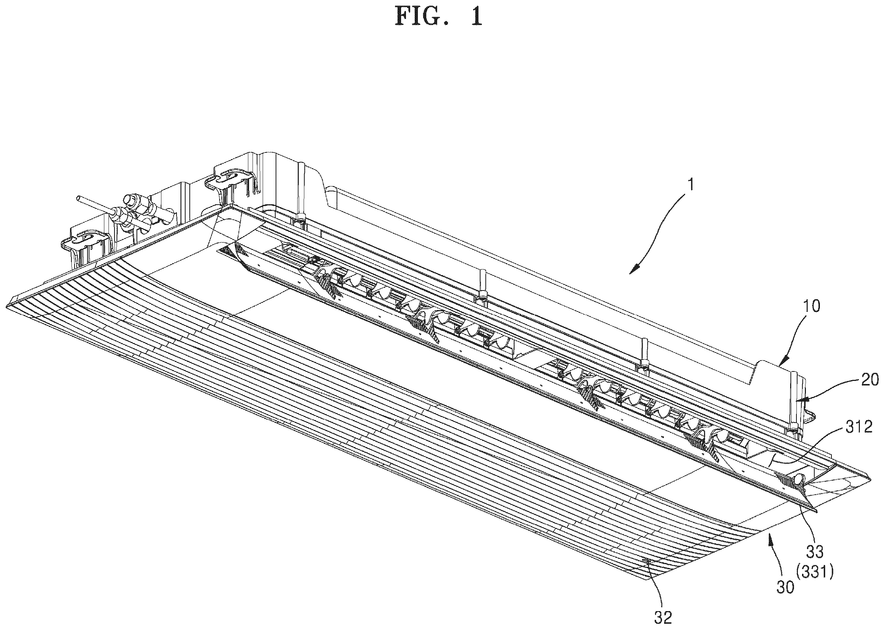

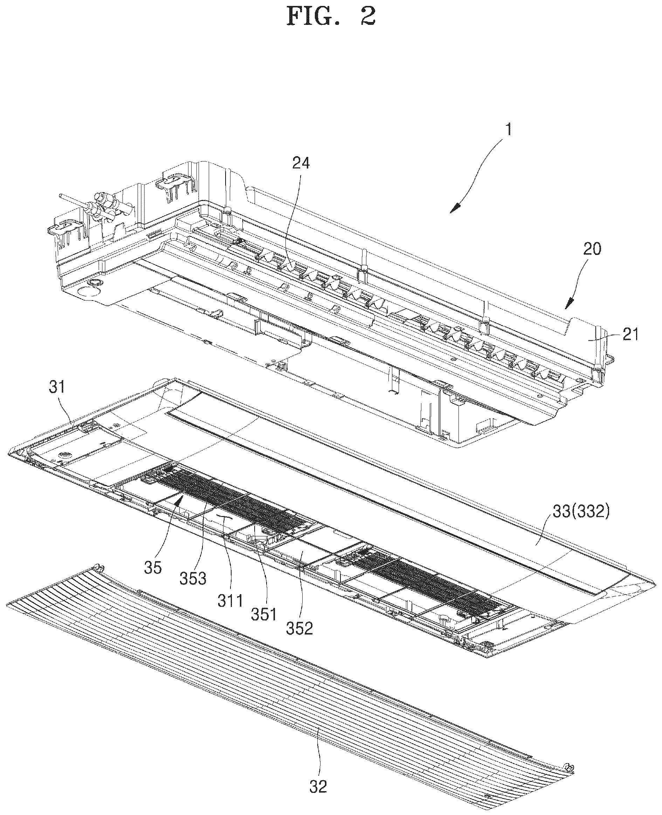

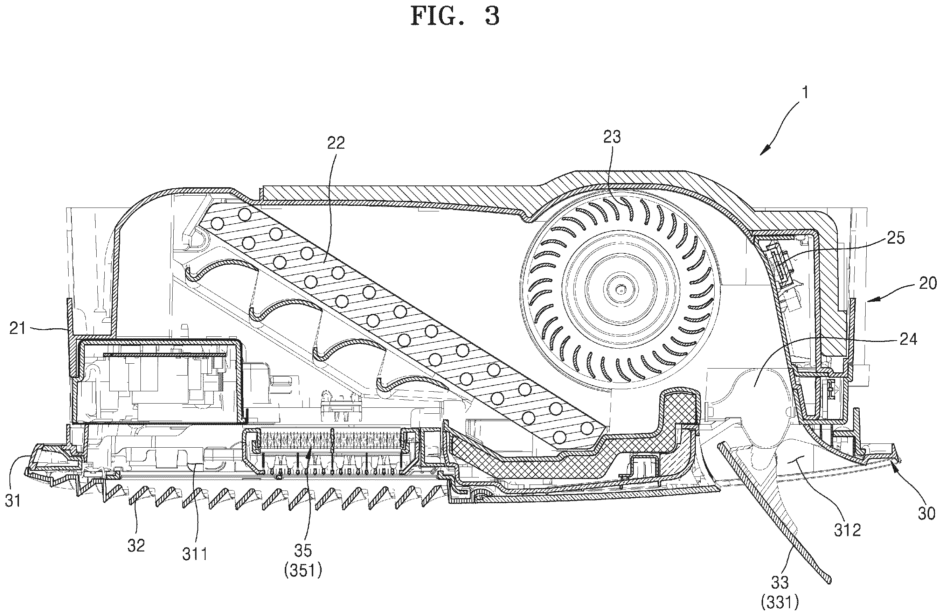

FIG. 1 is a perspective view of an air conditioner 1 according to various embodiments. FIG. 2 is an exploded perspective view of the air conditioner 1 according to various embodiments. FIG. 3 is a cross-sectional view of the air conditioner 1 according to various embodiments.

Referring to FIGS. 1, 2 and 3 (which may be referred to as FIGS. 1 to 3), according to an embodiment of the disclosure, the air conditioner 1 includes a main body 20 and a panel 30 assembled to the main body 20. A portion of the main body 20 and a portion of the panel 30 may form a housing 10.

The main body 20 may be installed into a ceiling. The air conditioner 1 may be a ceiling-mounted air conditioner. However, the main body 20 is not necessarily installed into the ceiling, and may be exposed to the outside as needed.

The main body 20 may include a main body frame 21, and a heat exchanger 22 and a blower fan 23 accommodated within the main body frame 21. The heat exchanger 22 may be the indoor heat exchanger as described above.

The heat exchanger 22 may be located or provided inside the housing 10 and induce heat exchange between air introduced into the main body frame 21 and a refrigerant. The heat exchanger 22 may be located in a flow path connecting between the air inlet 311 and the air outlet 312.

The blower fan 23 may be rotatably located inside the housing 10. The blower fan 23 may generate flow in a direction toward the air outlet 312. The blower fan 23 may be located between the heat exchanger 22 and the air outlet 312. In other words, the blower fan 23 may be located between the heat exchanger 22 and the flow path connecting the air outlet 312.

The plurality of wind direction guides 24 may control a direction of a wind discharged from the air outlet 312. The plurality of wind direction guides 24 may control the direction of the wind discharged from the air outlet 312 in a longitudinal direction Y of the housing 10. The plurality of wind direction guides 24 may control left and right directions of the wind discharged from the air outlet 312.

The plurality of wind direction guides 24 may be positioned between the blower fan 23 and the air outlet 312. The plurality of wind direction guides 24 may be arranged to be spaced apart along the longitudinal direction Y of the housing 10. The plurality of wind direction guides 24 may be rotatably mounted to the main body frame 21. The left and right directions of the discharged wind may vary depending on a rotation angle of the plurality of wind direction guides 24.

The panel 30 may include a panel frame 31 constructed to be assembled to the main body frame 21, and a grille panel 32 and a blade 33 installed on the panel frame 31.

The panel frame 31 may include an air inlet 311 via which air is drawn into the main body 20 and an air outlet 312 via which air is discharged to outside of the main body 20. The housing 10 may include the air inlet 311 and the air outlet 312. The panel frame 31 may be assembled to a bottom of the main body frame 21 to be detachable therefrom.

The grille panel 32 may be arranged to cover the air inlet 311. The grille panel 32 may include a plurality of grille holes provided to allow outside air to be drawn into the air inlet 311. The grille panel 32 may allow the outside air to enter the air inlet 311 via the grille holes while minimizing and/or reducing the unintentional introduction of foreign substances into the air inlet 311 of the panel frame 31.

The grille panel 32 may be assembled to one side of the panel frame 31 to be detachable therefrom. For example, the grille panel 32 may be rotatably assembled to the panel frame 31 and be affixed thereto using a hook. However, a method used to assemble the grille panel 32 is not limited thereto, and various other assembly methods may be used.

At least one filter 351 and 352 may be installed at the air inlet 311 of the panel frame 31. The filters 351 and 352 may be positioned between the main body 20 and the grille panel 32. The filters 351 and 352 may be assembled to the panel frame 31 to be detachable therefrom. For example, the filters 351 and 352 may be assembled into a filter frame 353, and the filter frame 353 may be mounted to the panel frame 31. In other words, a filter module 35 including the filters 351 and 352 and the filter frame 353 may be mounted to the panel frame 31. However, the mounting of the filters 351 and 352 is not limited thereto, and the filters 351 and 352 may be mounted directly to the panel frame 31 without the filter frame 353.

The filters 351 and 352 may include an electrostatic dust collection filter 351 that collects foreign substances using electrostatic forces. The filters 351 and 352 may further include a deodorizing filter 352 configured to remove odors. However, the types of filters 351 and 352 are not necessarily limited thereto, and may be modified in various ways as long as they are intended to adsorb or remove foreign substances from the air drawn in via the grille holes.

While the air conditioner 1 of FIGS. 2 and 3 has been described mainly with respect to an example in which the filters 351 and 352 are mounted to the panel frame 31, the filters 351 and 352 may be an optional component. For example, although not shown, the filters 351 and 352 may not be mounted to the panel frame 31 of the air conditioner 1.

The blade 33 may be installed to open and close the air outlet 312 of the panel frame 31. The blade 33 may be rotatably installed on the panel frame 31. The blade 33 may open or close the air outlet 312 by adjusting its rotation angle. The blade 33 may have an open pose 331 for opening the air outlet 312 and a closed pose 332 for blocking the air outlet 312. The term "pose" may be used interchangeably with the term "position" and is intended to cover a "position".

The blade 33 may control upward and downward directions of the wind discharged from the air outlet 312. Depending on the rotation angle of the blade 33, the upward and downward directions of the wind discharged from the air outlet 312 may vary. By adjusting the rotation angle of the blade 33, the direction and volume of wind discharged from the air outlet 312 may be adjusted.

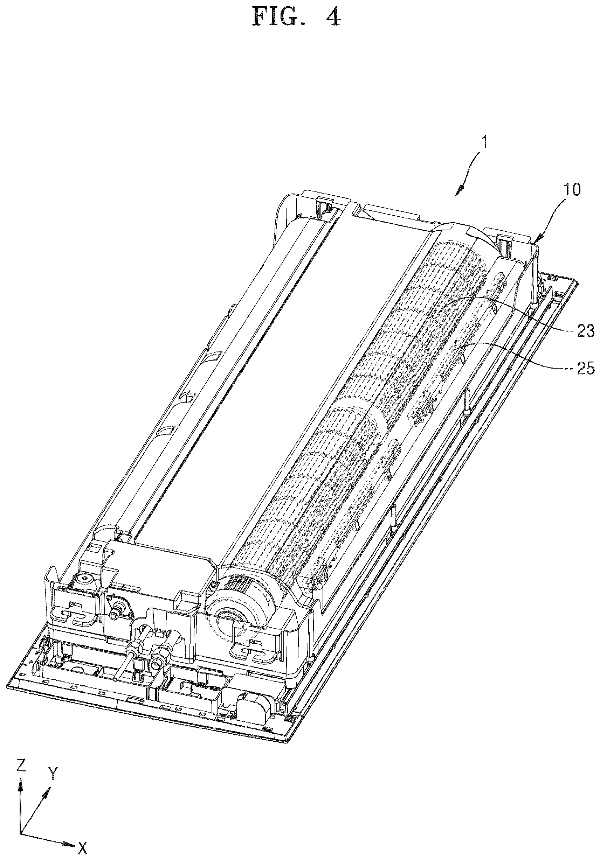

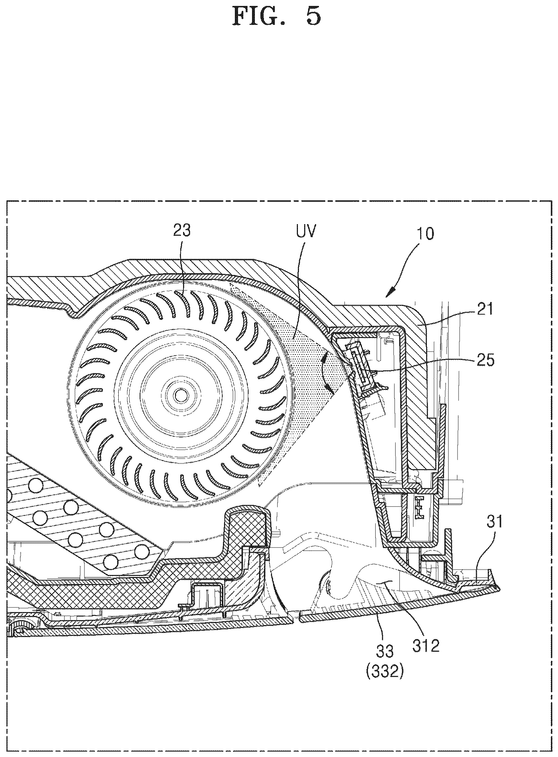

FIG. 4 is a perspective view illustrating the blower fan 23 and an ultraviolet (UV) light source 25 in the air conditioner 1, according to various embodiments. FIG. 5 is a partial cross-sectional view illustrating irradiation of the blower fan 23 with UV light by the UV light source 25 in the air conditioner 1, according to various embodiments.

Referring to FIG. 4, the blower fan 23 may extend along a direction perpendicular to a direction of movement of air toward the air outlet 312. The blower fan 23 may rotate about a rotation axis extending along the longitudinal direction Y of the housing 10. The blower fan 23 may be a cross flow fan. The longitudinal direction Y of the housing 10 may be perpendicular to a vertical direction Z of the housing 10 and a width direction X of the housing 10.

According to an embodiment of the disclosure, the air conditioner 1 may further include the UV light source 25 configured to irradiate UV light toward the blower fan 23. A wavelength of the UV light generated by the UV light source 25 may be a wavelength suitable for sterilization, for example, a UV-C wavelength. For example, the UV light may have a wavelength range of about 100 nanometers (nm) to about 280 nm.

The UV light source 25 may be located inside the housing 10. The UV light source 25 may be located inside the housing 10 to irradiate UV light toward the blower fan 23. The UV light source 25 may be located inside the main body frame 21. The housing 10 may include the main body frame 21, the panel frame 31, the grille panel 32, and an intermediate panel 34.

The UV light source 25 may be configured as a plurality of UV light sources 25. The plurality of UV light sources 25 may be arranged to be spaced apart along the longitudinal direction Y of the housing 10.

Referring to FIGS. 4 and 5, the UV light source 25 may be arranged to be spaced apart from the blower fan 23 along a radial direction of the blower fan 23. The blower fan 23 may be located within a UV irradiation range where UV light is irradiated from the UV light source 25.

An angle at which UV light is irradiated from the UV light source 25 toward the blower fan 23 may be an angle that covers a portion of the blower fan 23 facing the UV light source 25. The angle of the UV light (UV) irradiated from the UV light source 25 toward the blower fan 23 may be determined by taking into account a distance between the UV light source 25 and the blower fan 23 and a diameter of the blower fan 23.

The UV light source 25 may be arranged so that the UV light does not reach the air outlet 312. For example, the UV light source 25 may be positioned to face the blower fan 23 and be spaced apart from the air outlet 312 by a predetermined distance. The air outlet 312 may be located outside of the UV irradiation range of the UV light source 25.

A portion of the blower fan 23 that is directly irradiated with the UV light by the UV light source 25 may be a portion of a rim of the blower fan 23. In order to entirely irradiate the rim of the blower fan 23 with the UV light, the blower fan 23 may be rotated when the UV light is irradiated from the UV light source 25 toward the blower fan 23. In other words, while the blower fan 23 is rotating, the UV light may be irradiated toward the blower fan 23.

In order for the entire rim of the blower fan 23 to be sterilized by the UV light source 25, the blower fan 23 and the UV light source 25 need to operate for a certain amount of time. For example, to completely sterilize the rim of the blower fan 23, an operation time of the blower fan 23 and the UV light source 25 may be 1 hour or more . For example, the time required to sterilize the blower fan 23 may be 2 to 4 hours.

As described above, because the air outlet 312 is located outside the UV irradiation range of the UV light source 25, UV light is not exposed outside the air outlet 312 during normal use of the air conditioner 1. Accordingly, during normal use of the air conditioner 1, the UV light may not reach the user. However, when the blade 33 is in the open position 331, the user may insert a finger through the air outlet 312.

When a finger is inserted through the air outlet 312, the finger may reach the blower fan 23. For example, a distance between the air outlet 312 and the blower fan 23 may be 20 cm or less. For example, the distance between the air outlet 312 and the blower fan 23 may be 10 cm or less.

When a user's finger is inserted via the air outlet 312, the UV light irradiated toward the blower fan 23 may reach the user's finger. Here, the user may be a person who uses the air conditioner 1 as well as a person who repairs or installs the air conditioner 1.

In this way, when the UV light irradiated from the UV light source 25 reaches a part of a user's body, injury may occur to the user. In consideration of this, according to an embodiment, the air conditioner 1 may be configured to detect whether the air outlet 312 is accessible to the user (e.g., detect a position of the blade 33 described in greater detail below) and control operation of the UV light source 25 based on a detection result.

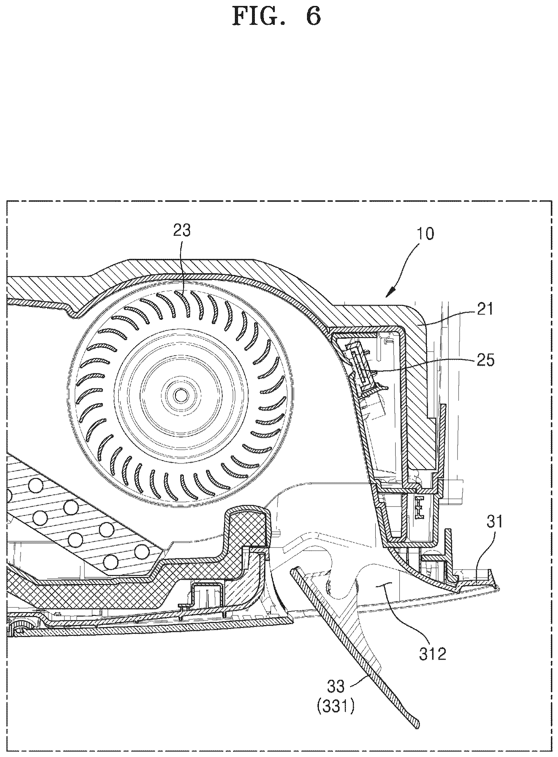

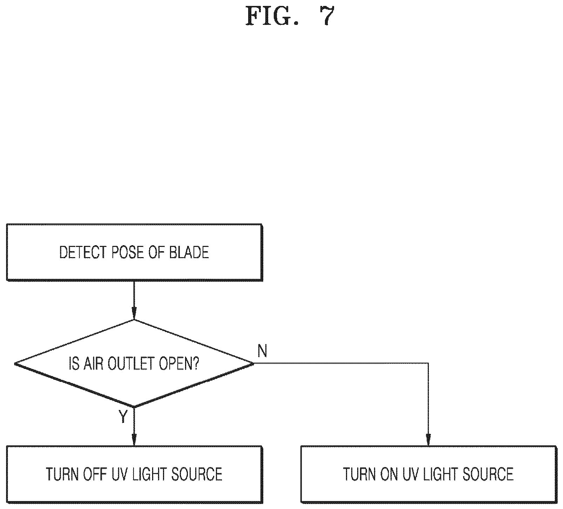

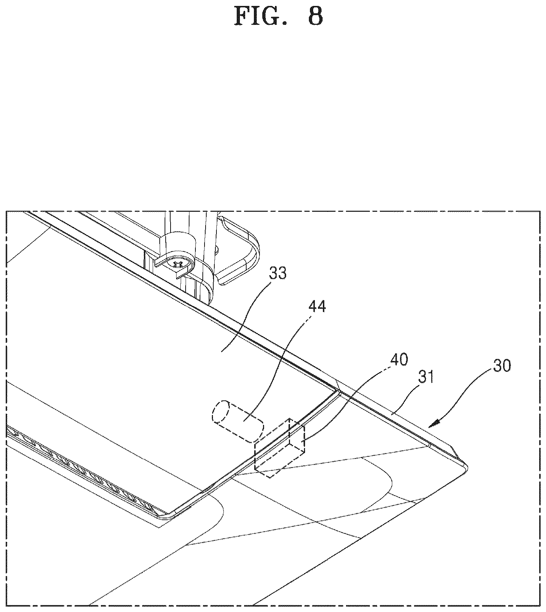

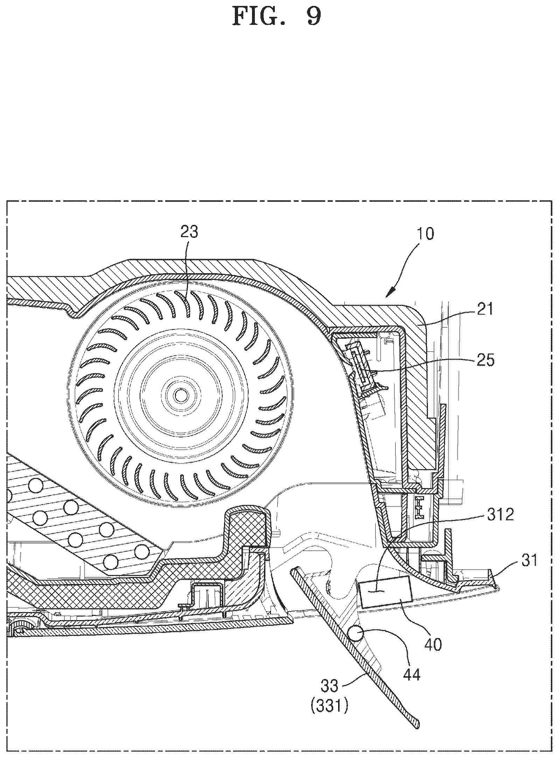

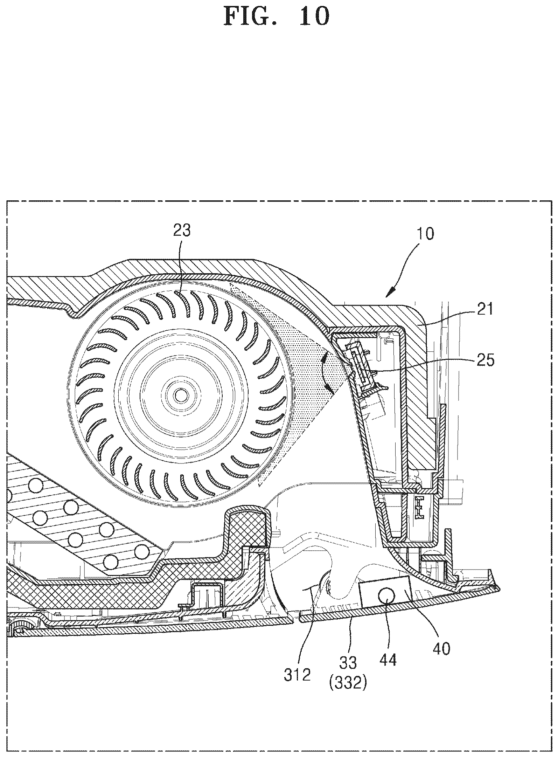

FIG. 6 is a cross-sectional view illustrating a state in which operation of the UV light source 25 of the air conditioner 1 is stopped, according to various embodiments. FIG. 7 is a flowchart illustrating an example operation of the UV light source 25 of the air conditioner 1, according to various embodiments. FIG. 8 is a perspective view illustrating an example of a sensor 40 of the air conditioner 1, according to various embodiments, and FIGS. 9 and 10 are diagrams illustrating operating states of the UV light source 25 using the sensor 40 of FIG. 8 according to various embodiments.

Referring to FIGS. 6 and 7, according to an embodiment of the disclosure, in the air conditioner 1, operation of the UV light source 25 may be selectively stopped based on a pose (e.g., position) of the blade 33. In other words, based on the position of the blade 33, the UV light source 25 may be selectively operated.

Based on the position of the blade 33, it may be determined whether the air outlet 312 is open. For example, when the blade 33 is in the closed position 332, it may be determined that the air outlet 312 is in a closed state. When the blade 33 is in the open position 331, it may be determined that the air outlet 312 is in an open state.

For example, the UV light source 25 may be operated only when the blade 33 is in the closed position 332 to block the air outlet 312. When the blade 33 is in the open position 331 to open the air outlet 312, operation of the UV light source 25 may be stopped. When the blade 33 is in the closed position 332 to block the air outlet 312, the operation of the UV light source 25 may be permitted.

When the blade 33 is in the open position 331, the user's finger may be inserted through the air outlet 312. However, because the operation of the UV light source 25 is stopped when the blade 33 is in the open position 331, a situation in which the finger is irradiated with UV light does not occur.

When the blade 33 is in the closed position 332, the finger is not irradiated with UV light because the air outlet 312 is blocked and the finger is not inserted through the air outlet 312.

According to an embodiment of the disclosure, the air conditioner 1 may include a sensor 40 configured to detect whether the air outlet 312 is accessible to the user. For example, the sensor 40 may be configured to detect a position of the blade 33 in order to detect whether the air outlet 312 is accessible to the user. The UV light source 25 may be selectively operated based on the position of the blade 33 detected by the sensor 40.

The sensor 40 may be configured to directly detect the position of the blade 33. For example, the sensor 40 may be positioned opposite to the blade 33. Based on a result of the detection by the sensor 40, it may be determined whether the air outlet 312 is open.

Referring to FIG. 8, according to an embodiment of the disclosure, the sensor 40 may include a magnetic sensor for detecting a position of the blade 33 using a magnetic force. For example, the sensor 40 may be one of, for example, and without limitation, a Hall-effect sensor, a magneto-resistive sensor, a magneto-inductive sensor, and a magnetically coupled sensor.

The sensor 40 may be installed on the panel frame 31 to face the blade 33. The sensor 40 may detect a magnet 44 provided on the blade 33. The sensor 40 may detect the position of the blade 33 by detecting a strength of a magnetic field generated by the magnet 44. However, arrangement of the sensor 40 and the magnet 44 is not limited thereto, and may vary. For example, the sensor 40 may be disposed on the blade 33 and the magnet 44 may be disposed on the panel frame 31.

According to an embodiment of the disclosure, in the air conditioner 1, when the magnet 44 on the blade 33 moves away from the sensor 40, as shown in FIG. 9, the sensor 40 may detect that the blade 33 is in the open position 331. Based on this detection result, it may be determined that the air outlet 312 is in an open state. It may be determined by the sensor 40 that the air outlet 312 is accessible to the user. When the blade 33 is in the open position 331 to open the air outlet 312, the operation of the UV light source 25 may be stopped. In other words, when the blade 33 is in the open position 331 to open the air outlet 312, the UV light source 25 may not operate.

According to an embodiment of the disclosure, in the air conditioner 1, when the sensor 40 and the magnet 44 on the blade 33 approach each other, as shown in FIG. 10, the sensor 40 may detect that the blade 33 is in the closed position 332. For example, when the sensor 40 and the magnet 44 on the blade 33 overlap, the sensor 40 may detect that the blade 33 is in the closed position 332. Based on this detection result, it may be determined that the air outlet 312 is in a closed state. When the blade 33 is in the closed position 332 to block the air outlet 312, the operation of the UV light source 25 may be permitted. In other words, when the blade 33 is in the closed position 332 to block the air outlet 312, the UV light source 25 may be operated.

While the sensor 40 using a magnetic force is used in the above-described embodiment of the disclosure, a type of the sensor 40 is not limited thereto and may vary.

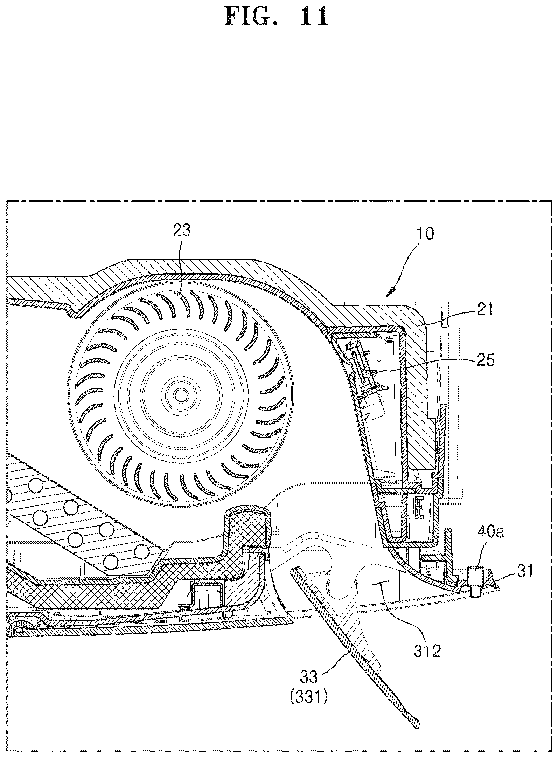

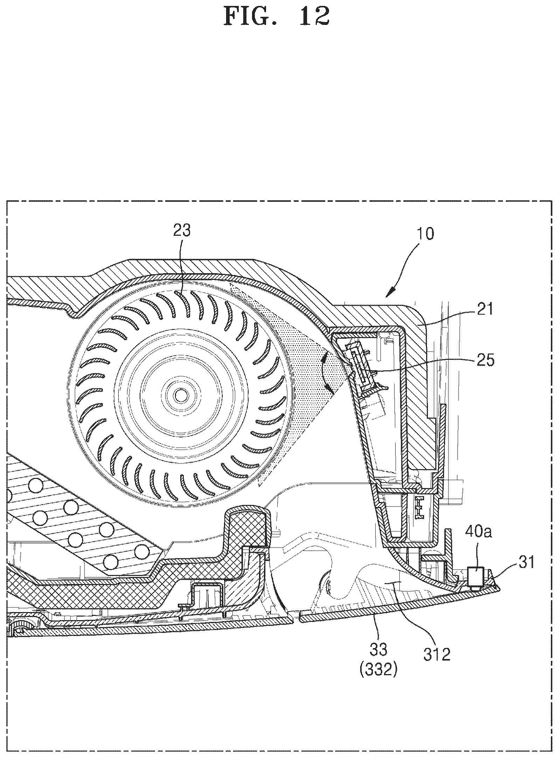

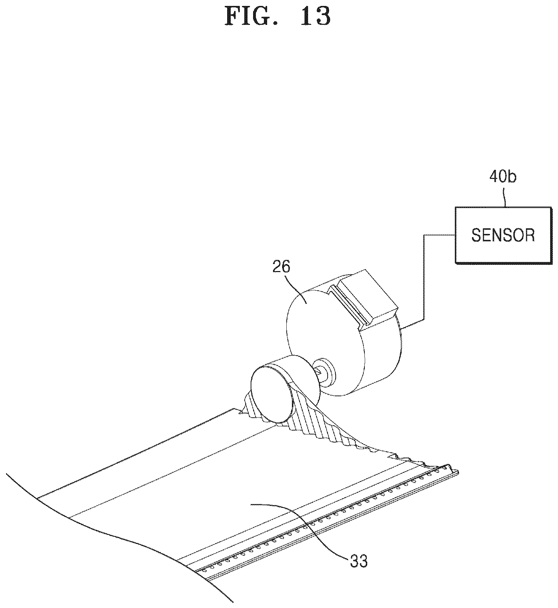

FIGS. 11 and 12 are cross-sectional views illustrating a sensor 40a, which is another example of the sensor 40, of the air conditioner 1, according to various embodiments. FIG. 13 is a diagram illustrating a sensor 40b, which is another example of the sensor 40, of the air conditioner 1, according to various embodiments.

Referring to FIGS. 11 and 12, according to an embodiment of the disclosure, the sensor 40a may be arranged opposite to the blade 33. The sensor 40a may be a contact sensor whose contact with the blade 33 varies depending on the position of the blade 33. For example, the sensor 40a may be a sensor that is contacted and pressed by the blade 33 when the blade 33 is in the closed position 332.

According to an embodiment of the disclosure, in the air conditioner 1, when the blade 33 is not in contact with the sensor 40a, as shown in FIG. 11, the sensor 40a may detect that the blade 33 is in the open position 331. Based on this detection result, it may be determined that the air outlet 312 is in an open state. It may be determined by the sensor 40a that the UV light source 25 is accessible to the user. When the blade 33 is in the open position 331 to open the air outlet 312, the operation of the UV light source 25 may be stopped. In other words, when the blade 33 is in the open position 331 to open the air outlet 312, the UV light source 25 may not operate.

According to an embodiment of the disclosure, in the air conditioner 1, when the sensor 40a is contacted and pressed by the blade 33, as shown in FIG. 12, the sensor 40 may detect that the blade 33 is in the closed position 332. Based on this detection result, it may be determined that the air outlet 312 is in a closed state. When the blade 33 is in the closed position 332 to block the air outlet 312, operation of the UV light source 25 may be permitted. In other words, when the blade 33 is in the closed position 332 to block the air outlet 312, the UV light source 25 may be operated.

According to an embodiment of the disclosure, the sensor 40b may be configured to indirectly detect a position of the blade 33. For example, the sensor 40b may indirectly detect the position of the blade 33 via surrounding components of the blade 33.

For example, as shown in FIG. 13, the sensor 40b may be a sensor for detecting a rotation angle of a driver 26 that rotates the blade 33. The sensor 40b may detect the position of the blade 33 by detecting the rotation angle of the driver 26. When the closed position 332 of the blade 33 is detected by the sensor 40b, operation of the UV light source 25 may be permitted. When the open position 331 of the blade 33 is detected by the sensor 40b, operation of the UV light source 25 may be stopped.

The types of sensors 40, 40a, and 40b according to an embodiment of the disclosure are examples, and the types and arrangements of the sensors 40, 40a, and 40b may be modified in various ways as long as the sensors are intended to directly or indirectly detect the pose (position) of the blade 33.

In the air conditioner 1 according to an embodiment of the disclosure, when the blade 33 is in the closed position 332, UV light may be irradiated from the UV light source 25 while the blower fan 23 is operating. However, when the blower fan 23 operates while the blade 33 is in the closed position 332, but air is not discharged to the outside of the housing 10, a overcooling state may occur in which an internal temperature of the housing 10 drops to a predetermined temperature or lower. Due to the overcooling state, ice may appear on an inside of the housing 10.

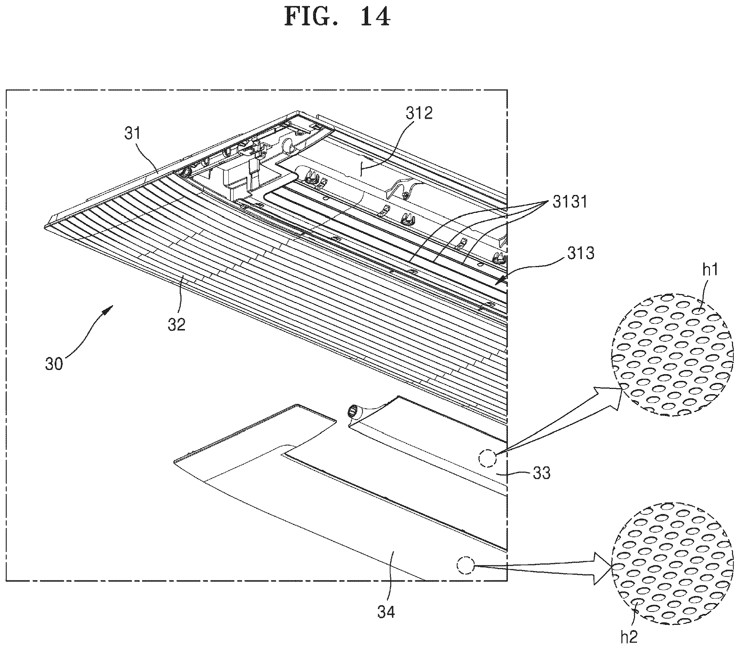

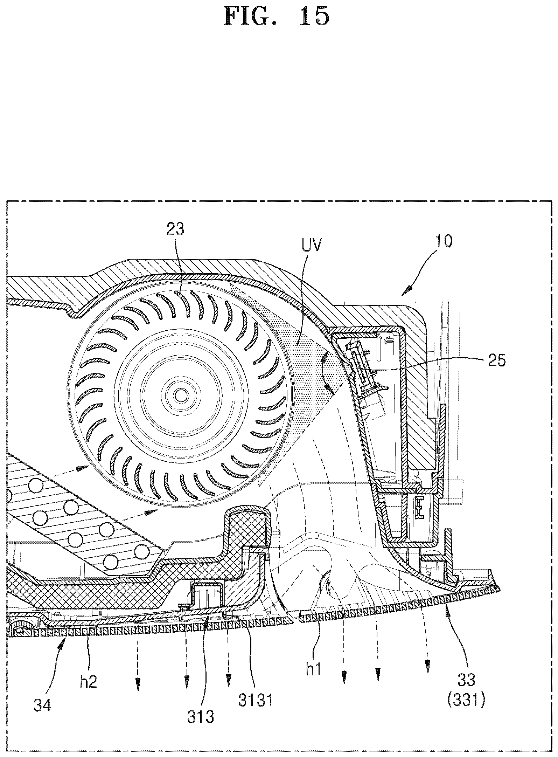

FIG. 14 is a partial perspective view illustrating various components surrounding the air outlet 312 of the air conditioner 1, according to various embodiments. FIG. 15 is a cross-sectional view illustrating an example in which air is discharged to outside of the housing 10 when the blade 33 is in the closed position 332 in the air conditioner 1, according to various embodiments.

Referring to FIGS. 14 and 15, according to an embodiment of the disclosure, the air conditioner 1 may be configured to allow air to be discharged when the blade 33 is in the closed position 332. As an example for this configuration, the blade 33 may include a plurality of first holes h1. Each of the plurality of first holes h1 may be of a size that allows air to be discharged therethrough but does not allow a user's finger to be inserted thereinto. For example, each of the plurality of first holes h1 may have a size of about 0.1 millimeters (mm) to about 10 mm. For example, each of the plurality of first holes h1 may have a size of 5 mm or less. For example, each of the plurality of first holes h1 may have a size of 3 mm or less.

The housing 10 of the air conditioner 1 may include an intermediate panel 34 mounted on the panel frame 31 so as to be located between the grille panel 32 and the blade 33. The intermediate panel 34 may cover an intermediate portion 313 that surrounds the air outlet 312 in the panel frame 31.

The intermediate panel 34 may be assembled to the panel frame 31 to be detachable therefrom. For example, the grille panel 32 may be assembled to the panel frame 31 using, for example, a hooking method. However, a form in which the intermediate panel 34 is assembled is not limited thereto, and may vary.

The intermediate panel 34 may include a plurality of second holes h2 through which air may be discharged. Each of the plurality of second holes h2 may be of a size that allows air to be discharged therethrough but does not allow a user's finger to be inserted thereinto. For example, each of the plurality of second holes h2 may have a size of about 0.1 mm to about 10 mm. For example, each of the plurality of second holes h2 may have a size of 5 mm or less. For example, each of the plurality of second holes h2 may have a size of 5 mm or less. The size of each of the second holes h2 may correspond to the size of each of the first holes h1.

Air discharged from the air outlet 312 may be delivered to the intermediate portion 313 of the panel frame 31 covered by the intermediate panel 34. A plurality of ribs 3131 may be arranged in the intermediate portion 313 of the panel frame 31 to change a direction of the air delivered from the air outlet 312. The plurality of ribs 3131 may extend in a direction perpendicular to the direction of air movement, e.g., along the longitudinal direction of the housing 10. The air whose direction has been changed by the plurality of ribs 3131 may be discharged to the outside through the second holes h2.

While the above example embodiment of the disclosure has been described with respect to an example in which the plurality of second holes h2 are formed in the intermediate panel 34, it is not necessarily limited thereto. For example, the intermediate panel 34 may not include the second holes h2. In this case, when the blade 33 is in the closed position 332, air may be discharged through the plurality of first holes h1.

In addition, the air conditioner 1 according to various embodiments of the disclosure has been described with respect to an example in which the UV light source 25 is selectively operated by determining whether the air outlet 312 is in an open state based on the position of the blade 33. However, the selective operation of the UV light source 25 in the air conditioner 1 according to an embodiment of the disclosure is not necessarily limited to this example, and may be variously modified as long as a corresponding configuration is for detecting whether the air outlet 312 is accessible to the user to prevent and/or reduce exposure of the user to UV light.

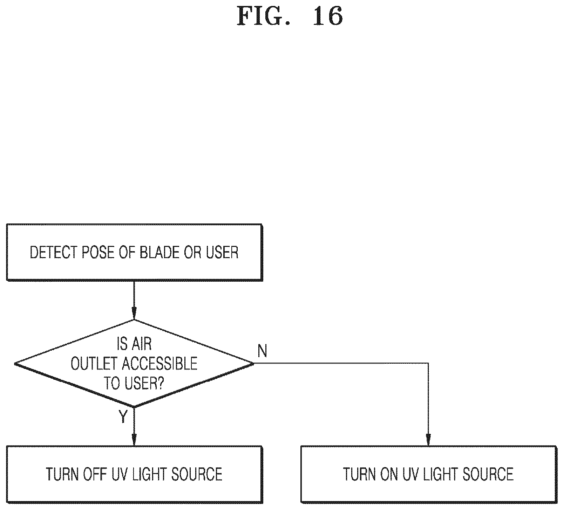

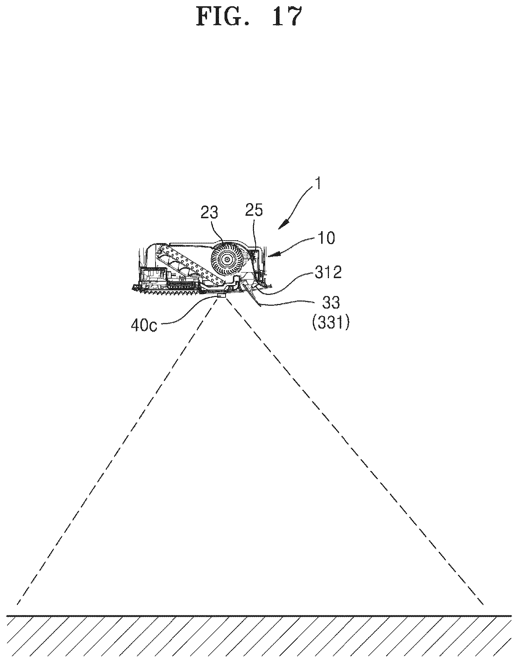

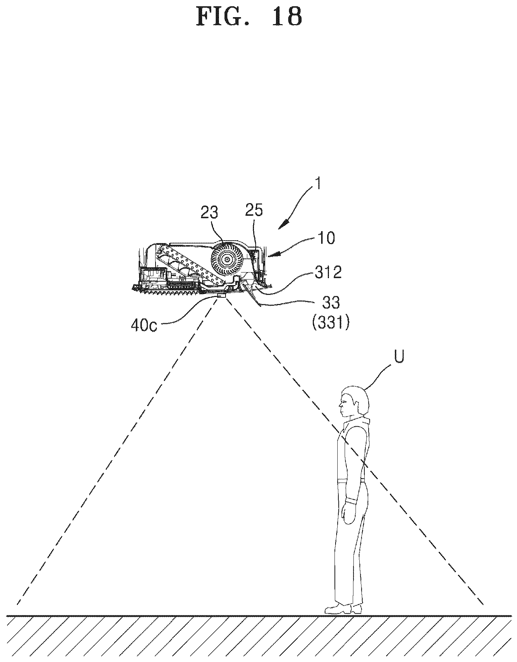

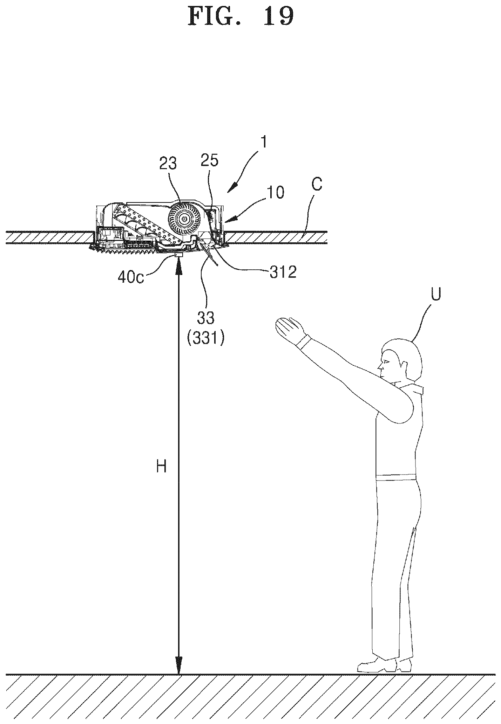

FIG. 16 is a flowchart illustrating example operation of the UV light source 25 in the air conditioner 1, according to various embodiments, and FIGS. 17 and 18 are diagrams illustrating whether the UV light source 25 of the air conditioner 1 is operating, according to various embodiments. FIG. 19 is a diagram illustrating an example reference distance between a user U and a sensor 40c in the air conditioner 1, according to various embodiments.

Referring to FIG. 16, according to an embodiment of the disclosure, the air conditioner 1 may include the sensor 40 that detects a pose(or position) of the blade 33 or the user. The sensor 40 may directly or indirectly detect whether the air outlet 312 is accessible to the user.

For example, the sensor 40 may indirectly detect whether the air outlet 312 is accessible to the user. For example, as described above with reference to FIGS. 8 to 13 in various embodiments of the disclosure, the sensors 40, 40a, and 40b may be sensors that detect the position of the blade 33. The sensor 40, 40a, or 40b may detect whether the blade 33 is in the open position 331. Based on a result of the detection by the sensor 40, 40a, or 40b, the air conditioner 1 may determine whether the user has access the air outlet 312.

In another example, the sensor 40c may be configured to detect a user. For example, the sensor 40c may detect the presence or absence of a user or a user's location. The sensor 40c may directly detect whether the air outlet 312 is accessible to the user. For example, the sensor 40c may be a human detection sensor. For example, the sensor 40c may be an infrared sensor or a radar sensor.

For example, referring to FIGS. 17 and 18, the sensor 40c may detect whether the user U is present within a certain space (e.g., illustrated by the dotted lines). The air conditioner 1 may detect, via the sensor 40c, whether the user U is present within the certain space, thereby determining whether the air outlet 312 is accessible to the user U.

For example, as shown in FIG. 17, when the sensor 40c does not detect the user U, the air conditioner 1 may determine that the air outlet 312 is not accessible to the user U. Accordingly, the air conditioner 1 may operate the UV light source 25 in conjunction with operation of the blower fan 23 when the blade 33 is in the open position 331. A process of sterilizing the blower fan 23 may be performed using UV light generated by the UV light source 25.

For example, as shown in FIG. 18, when the sensor 40c detects the user U, the air conditioner 1 may determine that the air outlet 312 is accessible to the user U. Accordingly, the air conditioner 1 may not operate the UV light source 25. As a result, the user U may be prevented and/or inhibited from being injured by UV light.

For efficient operation of the UV light source 25, the sensor 40c may detect a direction of the user U and a distance from the sensor 40c to the user U. The sensor 40c may be a radar sensor. The sensor 40c may detect whether the distance to the user U has narrowed to within a predetermined reference distance, and the air conditioner 1 may determine, based on a detection result, whether the air outlet 312 is accessible to the user U.

Referring to FIG. 19, the reference distance between the sensor 40c and the user U may be determined by taking into account an installation height H of the air conditioner 1, an average height of an adult male, and an average arm length of the adult male. The reference distance between the sensor 40c and the user U may take into account a distance between the air outlet 312 and fingertips of the user U when the user U extends his or her arm upward. For example, the reference distance between the sensor 40c and the user U may be a distance of 1.0 m or more between the air outlet 312 and the head of the user U. The reference distance between the sensor 40c and the user U may be a distance of 1.3 m or more between the air outlet 312 and the head of the user U. The reference distance between the sensor 40c and the user U may be a distance of 1.5 m or more between the air outlet 312 and the head of the user U. The reference distance between the sensor 40c and the user U may be a distance of 10 m or less between the air outlet 312 and the head of the user U.

Moreover, the above example embodiment of the disclosure has been described with respect to a ceiling-mounted air conditioner installed on a ceiling C. However, according to an embodiment of the disclosure, the air conditioner 1 may be applied in various ways as long as it has a structure that includes the UV light source 25 for sterilizing the blower fan 23 inside the housing 10.

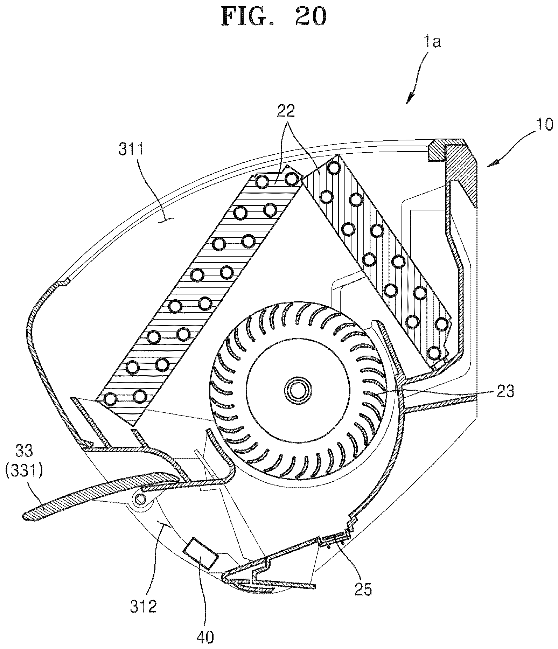

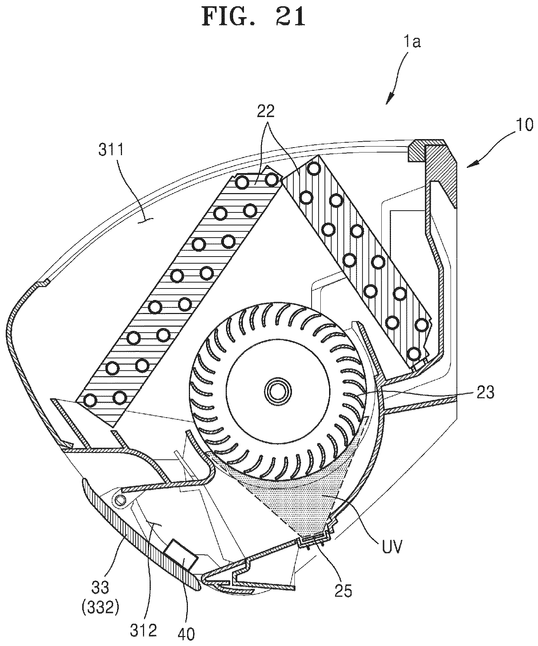

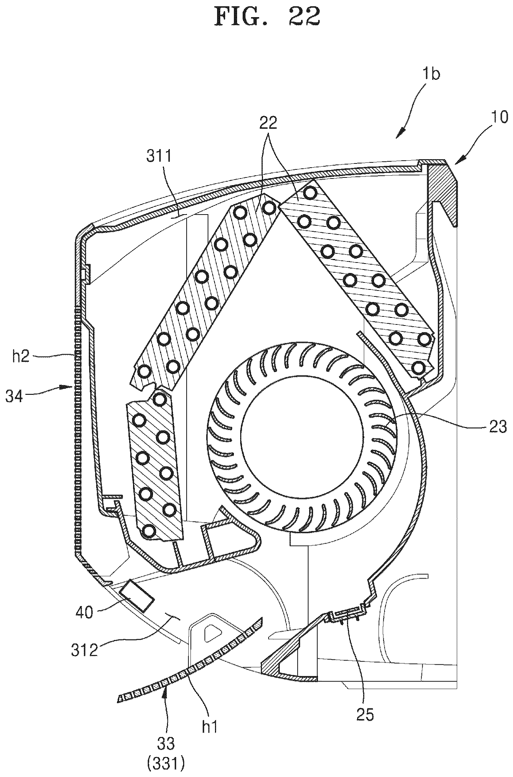

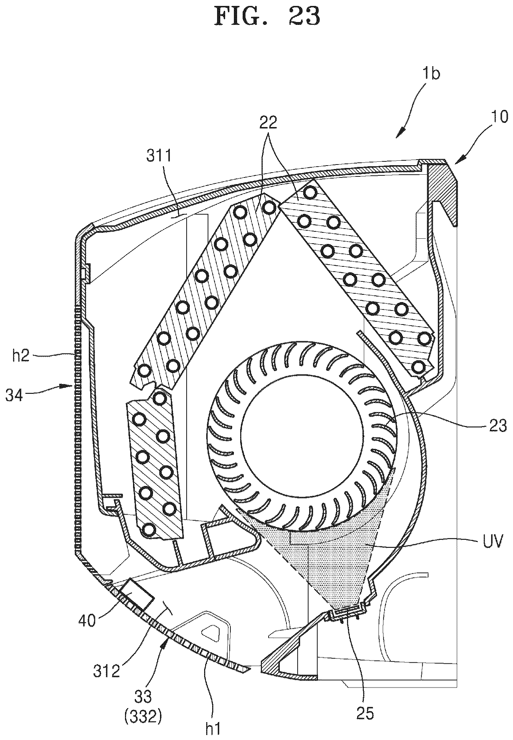

FIG. 20 is a cross-sectional view illustrating an example of an air conditioner 1a according to various embodiments, and FIG. 21 is a cross-sectional view illustrating example operation of the air conditioner 1a of FIG. 20 according to various embodiments. FIG. 22 is a cross-sectional view illustrating an example of an air conditioner 1b according various embodiments, and FIG. 23 is a cross-sectional view illustrating example operation of the air conditioner 1b of FIG. 22 according to various embodiments.

Referring to FIGS. 20 and 21, the air conditioner 1a may be a wall-mounted air conditioner that may be installed on a wall.

According to an embodiment of the disclosure, the air conditioner 1a may include a housing 10 having an air inlet 311 and an air outlet 312, a blower fan 23 located inside the housing 10, a heat exchanger 22 located inside the housing 10, a UV light source 25 that irradiates UV light toward the blower fan 23, a blade 33 that opens and closes the air outlet 312, and a sensor 40 that detects a position of the blade 33.

The UV light source 25 may be selectively operated based on the position of the blade 33 detected by the sensor 40. The UV light source 25 may be permitted to operate when the blade 33 is in the closed position 332.

The blade 33 may include a plurality of first holes (not shown). Accordingly, when the blade 33 is in the closed position 332, air may be discharged through the plurality of first holes.

Referring to FIGS. 22 and 23, according to an embodiment of the disclosure, the air conditioner 1b may include an intermediate panel 34 located adjacent to the blade 33 in the housing 10. The intermediate panel 34 may include a plurality of second holes h2. Accordingly, operation of the UV light source 25 may be permitted when the sensor 40 detects that the blade 33 is in the closed position 332. When the blade 33 is in the closed position 332, air may be discharged through the plurality of first holes h1 in the blade 33 and the second holes h2 in the intermediate panel 34.

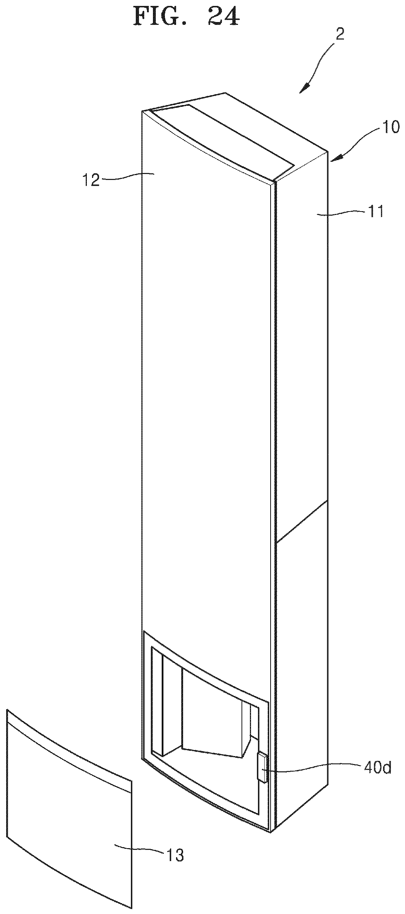

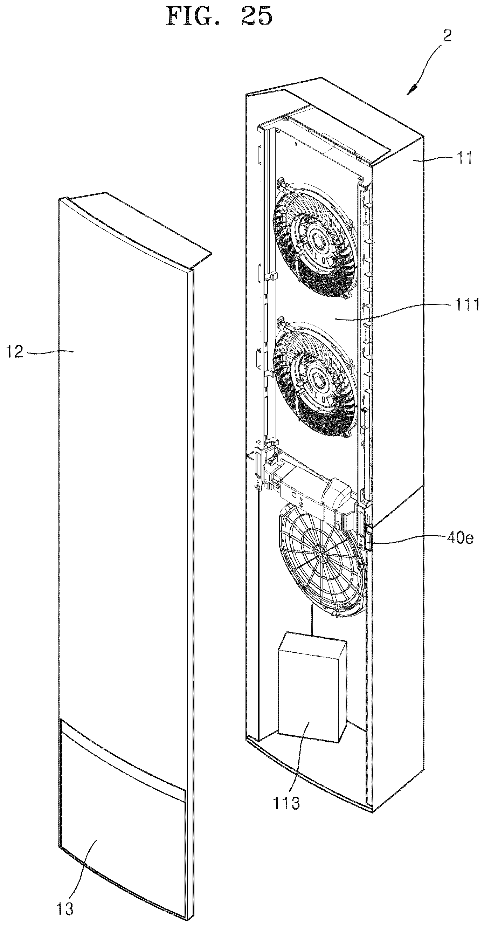

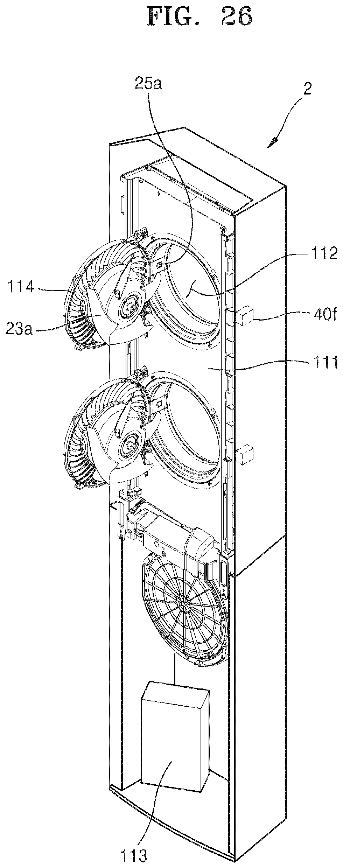

FIG. 24 is a perspective view of an air conditioner 2 according to various embodiments, and FIG. 25 is an exploded perspective view of the air conditioner 2 according to various embodiments, illustrating a front panel 12 detached from the air conditioner 2. FIG. 26 is a perspective view of the air conditioner 2 according to various embodiments, which illustrates the front panel 12 removed and a blower fan assembly detached from the air conditioner 2.

Referring to FIGS. 24, 25 and 26 (which may be referred to as FIGS. 24 to 26), according to an embodiment of the disclosure, the air conditioner 2 may be a stand-type air conditioner. According to an embodiment of the disclosure, the air conditioner 2 may include a housing 10 having an air inlet (not shown) and an air outlet (not shown), a blower fan 23a located inside the housing 10, a heat exchanger (not shown) located inside the housing 10, a UV light source 25a that irradiates UV light toward the blower fan 23a, and at least one sensor 40d, 40e, and 40f for detecting whether the UV light source 25a is accessible to a user.

The housing 10 may include a rear housing 11 and a front panel 12 detachably assembled on a front side of the rear housing 11. The front panel 12 may include a plurality of holes through which air is discharged toward the front.

At least one blower fan 23a, an internal frame 111 supporting the blower fan 23a, and a control box 113 for controlling the air conditioner 2 may be arranged inside the rear housing 11.

A lower cover 13 configured to expose a portion of an internal structure of the rear housing 11 may be provided at a lower portion of the front panel 12. The lower cover 13 may be detachably assembled to the front panel 12. When the lower cover 13 is removed, a portion of an inner space of the rear housing 11 in which the control box 113 is located may be exposed.

The internal frame 111 includes at least one fan receiving portion 112 accommodating the blower fan 23a. A fan grille 114 provided in front of the blower fan 23a may be disposed on the internal frame 111. The blower fan 23a may be supported to be rotatable about a predetermined hinge axis relative to the internal frame 111. The blower fan 23a may be rotated between a state in which it is inserted into the fan receiving portion 112 and a state in which it is detached from the fan receiving portion 112. The fan grille 114 and the blower fan 23a as a single module may be supported to be rotatable about the predetermined hinge axis. The UV light source 25a for sterilizing the blower fan 23a may be located in the fan receiving portion 112.

The sensors 40d, 40e, and 40f may be configured to detect at least one of whether the front panel 12 is detached, whether the lower cover 13 is detached, or whether the blower fan 23a is detached.

For example, as shown in FIG. 24, the sensor 40d may be installed around a lower opening of the front panel 12. Accordingly, the sensor 40d may detect whether the lower cover 13 is detached to expose the lower opening of the front panel 12.

When the sensor 40d detects that the lower cover 13 is detached, it may be determined that the UV light source 25a of the air conditioner 2 is accessible to the user. When it is determined that the UV light source 25a is accessible to the user based on a result of the detection by the sensor 40d, the operation of the UV light source 25a may be stopped.

In another example, as shown in FIG. 25, the sensor 40e may be installed in the rear housing 11. Accordingly, the sensor 40e may detect whether the front panel 12 is detached from the rear housing 11.

When the sensor 40e detects that the front panel 12 is detached, it may be determined that the UV light source 25a of the air conditioner 2 is accessible to the user. When it is determined that the UV light source 25a is accessible to the user based on a result of the detection by the sensor 40e, the operation of the UV light source 25a may be stopped.

In another example, as shown in FIG. 26, the sensor 40f may be installed on the internal frame 111. The sensor 40f may detect whether the blower fan 23a is detached from the fan receiving portion 112.

When the sensor 40f detects that the blower fan 23a is detached from the fan receiving portion 23a, it may be determined that the UV light source 25a of the air conditioner 2 is accessible to the user. When it is determined that the UV light source 25a is accessible to the user based on a result of the detection by the sensor 40f, the operation of the UV light source 25a may be stopped.

The air conditioners 1, 1a, 1b, and 2 according to various embodiments of the disclosure have been described with respect to the structures and functions of the UV light sources 25 and 25a irradiating UV light toward the blower fans 23 and 23a. However, arrangements and functions of the UV light sources 25 and 25a are not necessarily limited thereto. For example, the UV light source 25 or 25a may irradiate UV light to components other than the blower fan 23 or 23a in the air conditioner 1, 1a, 1b, or 2.

Although reference has been made to embodiments of the disclosure illustrated in the drawings for understanding the disclosure, and specific terms have been used to describe various embodiments thereof, the scope of the disclosure is not limited by the specific terms, and the disclosure will be construed to encompass all embodiments that would normally occur to one of ordinary skill in the art.

Particular implementations described herein merely correspond to embodiments of the disclosure and do not limit the scope of the disclosure in any way. For the sake of brevity of the disclosure, descriptions of conventional electronic configurations, control systems, software, and other functional aspects of the systems may be omitted. Furthermore, connecting lines or connection members shown in various figures are intended to represent example functional connections and/or physical or logical couplings between components in the figures, and in an actual device, connections between components may be represented by many alternative or additional functional relationships, physical connections, or logical connections. In addition, an element may not be necessarily essential to the practice of the disclosure unless the element is specifically described as "essential," "critical," etc. As used herein, the terms such as "comprising", "including" and the like are used to be understood as being an open-ended term for describing embodiments of the disclosure.

The use of the terms "the" and similar referents in the context of describing the disclosure (especially in the context of the following claims) are to be construed to cover both the singular and the plural. Furthermore, recitation of ranges of values herein are merely intended to serve as a shorthand method of referring individually to each separate value falling within the range (unless otherwise indicated herein), and each separate value is incorporated into the disclosure as if it were individually recited herein. Lastly, operations of a method according to the disclosure described herein may be performed in any suitable order unless clearly specified herein or contradicted by context. The disclosure is not limited to the described order of the operations. The use of any and all examples or example language, e.g., "such as", etc., provided herein is merely intended to describe the disclosure in detail and does not impose a limitation on the scope of the disclosure unless otherwise limited by the claims. Furthermore, various changes and modifications will be readily apparent to one of ordinary skill in the art without departing from the spirit and scope of the disclosure.

According to an embodiment of the disclosure, an air conditioner may include a UV light source for sterilizing a blower fan, but in order to prevent and/or reduce injury to a user due to UV light, may control operation of the UV light source by detecting whether an air outlet through which air is discharged is accessible to the user.

According to an example embodiment of the disclosure, an air conditioner may include: a housing having an air inlet and an air outlet, a heat exchanger provided inside the housing, a blower fan rotatably provided inside the housing, a UV light source provided inside the housing and configured to irradiate UV light toward the blower fan, a blade rotatable relative to the housing and configured to have an open position for opening the air outlet and a closed position for blocking the air outlet, and a sensor configured to detect a position of the blade.

The air conditioner may selectively stop operation of the UV light source based on the position of the blade detected by the sensor.

When the blade is in the open position, the operation of the UV light source may be stopped, and when the blade is in the closed position, the operation of the UV light source may be permitted.

The blower fan may be configured to rotate about a rotation axis extending along a longitudinal direction of the housing, and the UV light source may be provided to be spaced apart from the blower fan along a radial direction of the blower fan.

When the blade is in the closed position, the UV light source may be configured to irradiate UV light toward the blower fan while the blower fan is rotating.

The blower fan may be provided within a UV irradiation range where UV light is irradiated from the UV light source, and the air outlet may be provided outside the UV irradiation range of the UV light source.

The blade may include a plurality of first holes, and when the blade is in the closed position, air may be discharged to outside of the housing through the first holes.

The housing may include a main body frame accommodating the blower fan and the heat exchanger therein, a panel frame configured to be assembled to the main body frame and having the air inlet and the air outlet, a grille panel mounted on the panel frame to cover the air inlet, and an intermediate panel mounted on the panel frame to be provided between the grille panel and the blade.

The intermediate panel may include a plurality of second holes, and when the blade is in the closed position, air may be discharged to outside of the housing through the second holes.

The sensor may be configured to directly detect the position of the blade, or may be configured to indirectly detect the position of the blade.

According to an example embodiment of the disclosure, an air conditioner may include: a housing having an air outlet configure to draw air in and an air outlet configured to discharge air, a heat exchanger provided inside the housing, a blower fan rotatably provided inside the housing, a UV light source provided inside the housing and configured to irradiate UV light toward the blower fan, a blade rotatable relative to the housing and configured to have an open position for opening the air outlet and a closed position for blocking the air outlet, and a sensor configured to detect whether the air outlet is accessible to a user.

The air conditioner may be configured to control operation of the UV light source based on a result of the detection by the sensor.

The sensor may be configured to detect a position of the blade, and based on a result of the detection by the sensor, the air conditioner may be configured to determine whether the air outlet is accessible to the user.

When the blade is in the closed position, it may be determined that the air outlet is inaccessible to the user, and the operation of the UV light source may be permitted.

The blade may include a plurality of first holes, and when the blade is in the closed position, air may be discharged to outside of the housing through the first holes.

The sensor may be configured to detect the user, and based on a result of the detection by the sensor, the air conditioner may be configure to determine whether the air outlet is accessible to the user.

When the user is detected by the sensor, it may be determined that the air outlet is accessible to the user, and the operation of the UV light source may be stopped, and when the user is not detected by the sensor, it may be determined that the air outlet is inaccessible to the user, and the operation of the UV light source may be permitted.

According to an embodiment of the disclosure, it is possible to efficiently sterilize the blower fan provided inside the housing while minimizing/reducing the risk of injury to the user due to UV light for sterilization.

While the disclosure has been illustrated and described with reference to various example embodiments, it will be understood that the various example embodiments are intended to be illustrative, not limiting. It will be further understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

## Claims

1. An air conditioner comprising:
a housing (10) including an air inlet (311) and an air outlet (312);
a heat exchanger (22) disposed inside the housing;
a blower fan (23) rotatably provided inside the housing;
an ultraviolet (UV) light source (25) provided inside the housing and configured to irradiate UV light toward the blower fan;
a blade (33), rotatable relative to the housing, and configured to be in an open position (331) to open the air outlet or in a closed position (332) to block the air outlet; and
a sensor (40) configured to detect a position of the blade,
wherein operation of the UV light source is selectively stopped based on the position of the blade detected by the sensor.

2. The air conditioner of claim 1, wherein,
based on the blade being in the open position, operation of the UV light source is configured to be stopped, and
based on the blade being in the closed position, operation of the UV light source is configured to be permitted.

3. The air conditioner of claim 1 or 2, wherein
the blower fan is configured to rotate about a rotation axis extending along a longitudinal direction of the housing, and
the UV light source is spaced apart from the blower fan along a radial direction of the blower fan.

4. The air conditioner of claim 3, wherein based on the blade being in the closed position, the UV light source is configured to irradiate UV light toward the blower fan while the blower fan is rotating.

5. The air conditioner of claim 3 or 4, wherein
the blower fan is provided within a UV irradiation range where UV light is irradiated from the UV light source, and
the air outlet is provided outside the UV irradiation range of the UV light source.

6. The air conditioner of any one of claims 1 to 5, wherein
the blade comprises a plurality of first holes (h1), and
based on the blade being in the closed position, the air conditioner is configured to discharge air to outside of the housing through the plurality of first holes.

7. The air conditioner of any one of claims 1 to 6, wherein
the housing comprises
a main body frame (21) accommodating the blower fan and the heat exchanger therein,
a panel frame (31) configured to be assembled to the main body frame and provided with the air inlet and the air outlet,
a grille panel (32) mounted on the panel frame and configured to cover the air inlet, and
an intermediate panel (34) mounted on the panel frame between the grille panel and the blade.

8. The air conditioner of claim 7, wherein
the intermediate panel comprises a plurality of second holes (h2), and
based on the blade being in the closed position, air is configured to be discharged to outside of the housing through the plurality of second holes.

9. The air conditioner of any one of claims 1 to 8, wherein the sensor (40; 40a; 40b) is configured to detect the position of the blade.

10. An air conditioner comprising:
a housing (10) including an air inlet (311) and an air outlet (312);
a heat exchanger (22) disposed inside the housing;
a blower fan (23) rotatably provided inside the housing;
an ultraviolet (UV) light source (25) provided inside the housing and configured to irradiate UV light toward the blower fan;
a blade (33) rotatable relative to the housing and configured to be in an open position (331) for opening the air outlet or in a closed position (332) for blocking the air outlet; and
a sensor (40; 40a; 40b; 40c) configured to detect whether the air outlet is accessible to a user,
wherein the air conditioner is configured to control operation of the UV light source based on a result of the detection by the sensor.

11. The air conditioner of claim 10, wherein
the sensor is configured to detect a position of the blade, and
based on a result of the detection by the sensor, the air conditioner is configured to determine whether the air outlet is accessible to the user.

12. The air conditioner of claim 11, wherein, based on the blade being in the closed pose, the air conditioner is configured to determine that the air outlet is inaccessible to the user, and permit operation of the UV light source.

13. The air conditioner of claim 12, wherein
the blade comprises a plurality of first holes (h1) , and
based on the blade being in the closed position, air is configured to be discharged to outside of the housing through the plurality of first holes.

14. The air conditioner of any one of claims 10 to 13, wherein
the sensor is configured to detect the user, and
based on a result of the detection by the sensor, the air conditioner is configured to determine whether the air outlet is accessible to the user.

15. The air conditioner of claim 14, wherein,
based on the user being detected by the sensor, the air conditioner is configured to determine that the air outlet is accessible to the user, and stop operation of the UV light source, and
based on the user not being detected by the sensor, the air conditioner is configured to determine that the air outlet is inaccessible to the user, and permit operation of the UV light source.
